# EUROPÄISCHE PATENTSCHRIFT

(11) **EP 1 164 935 B1**
(45) Veröffentlichungstag und Bekanntmachung des Hinweises auf die Patenterteilung: **31.10.2007**
(21) Anmeldenummer: 00922446.0
(22) Anmeldetag: 22.03.2000
(51) Int. Cl.: A61B 6/14

(54) **VERFAHREN ZUR ERSTELLUNG UND WIEDERGABE EINES SCHICHTBILDES EINES OBJEKTS AUF EINER RÖNTGENSCHICHTAUFNAHME**
METHOD FOR MAKING AND REPRODUCING A TOMOGRAM OF AN OBJECT, SAID TOMOGRAM PERTAINING TO A SECTION ROENTGENOGRAM
PROCEDE PERMETTANT DE GENERER ET DE RESTITUER UNE COUPE D'UN OBJET A PARTIR D'UNE TOMOGRAPHIE

(30) Priorität: 22.03.1999 DE 19912854
(43) Veröffentlichungstag der Anmeldung: 02.01.2002
(73) Patentinhaber: Sirona Dental Systems GmbH, 64625 Bensheim (DE)
(72) Erfinder: DÖBERT, Michael, D-64653 Lorsch (DE); BONK, Roland, 67575 Eich (DE)
(74) Vertreter: Sommer, Peter
(86) Internationale Anmeldenummer: PCT/DE2000/000867
(87) Internationale Veröffentlichungsnummer: WO 2000/056217

(56) Entgegenhaltungen:
- EP-A- 0 000 079
- DE-A- 19 619 925
- DE-A- 19 733 338

## Beschreibung

### Technisches Gebiet

Die Erfindung bezieht sich auf ein Verfahren zur Erstellung und Wiedergabe eines Schichtbildes eines-Objekts aus einer Röntgenschichtaufnahme, insbesondere bei der Abbildung von Röntgenstrahlung auf einem digitalen Aufnahmesystem zur Erzeugung von Schichtaufnahmen, insbesondere von Panoramaschichtaufnahmen.

Bei Röntgenaufnahmen, auch bei digitalen Röntgenaufnahmen, verläuft der Strahlengang ausgehend von einer Strahlenquelle, dem Fokus, durch das zu durchstrahlende Objekt, in dem die Fokalebene liegt, beispielsweise Ober- oder Unterkieferbereiche, bevor die Strahlen auf die Sensorebene treffen, in der das Objekt abgebildet wird. Der Abstand einerseits zwischen der Fokalebene und dem Fokus und andererseits zwischen der Fokalebene und der Sensor- oder Empfangsebene ergibt sich in der Regel aus den Abmessungen des zu durchstrahlenden Objekts und aus den Aufnahmeverhältnissen.

Der Strahlengang von der Strahlenquelle durch die Fokalebene in die die Strahlung empfangende Ebene folgt den Gesetzmäßigkeiten des Strahlensatzes. Ausgehend von der Strahlungsquelle verläuft das Strahlenbündel durch die Fokalebene, die sich in dem abzubildenden Objekt befindet. In der Fokalebene hat das Objekt die wahre Größe, im Maßstab von 1:1. Von der Objektebene aus weitet sich das Strahlenbündel - nunmehr in Stärke und Kontrast durch das Objekt beeinflusst - weiter auf, wodurch das Objekt der Fokalebene in vertikaler und horizontaler Richtung abhängig vom Abstand der Fokalebene von der Sensor- oder Empfangsebene vergrößert und damit verzerrt wird. Das Objekt, beispielsweise einzelne Zähne oder ganze Partien von Ober- bzw. Unterkiefer, werden in der Empfangsebene um einen zwischen 1,05 und 1,24 liegenden Faktor in vertikale Richtung gesehen, vergrößert abgebildet. Ohne Korrektur entsteht bei Röntgenaufnahmen, auch bei digitalen Röntgenaufnahmen, eine verzerrte Darstellung des Objekts auf der Abbildungsebene.

Bei der Diagnose eines solchen Röntgenbildes und der Auswertung von sich ergebenden Besonderheiten auf dem Röntgenbild, muss der vertikalen und horizontalen Verzerrung einzelner Bereiche des Röntgenbildes Rechnung getragen werden. Zum Erreichen maßstabsgerechter Verhältnisse auf dem Röntgenbild müssen die verzerrten Bereiche des Röntgenbildes visuell interpoliert werden. Dies kann zu Fehlern bei der Interpretation führen, insbesondere aus der oft schwer einschätzbaren räumlichen Zuordnung.

### Stand der Technik

Bisher hat man sich damit beholfen, die tatsächlichen Größenverhältnisse des zu diagnostizierenden Bereiches aus dem Film oder aus dem Ausdruck bei digitalen Röntgenaufnahmen mit Hilfe von Rückrechnungen, Schablonen oder aus dem Strahlensatz bei bekanntem Vergrößerungsfaktor zwischen Objekt und Empfangsebene zu bestimmen.

Aus der DE 196 19 925 A1 ist bekannt, bei Panorama-Röntgengeräten zur Erstellung von Schichtaufnahmen die Position und räumliche Orientierung des Untersuchungsobjekts und deren Änderungen während des Aufnahmeablaufs durch Messung mittels eines Positionsdedektors zu erfassen. Aus den Signalen des Positionsdedektors werden dann Korrektursignale gebildet, um vor der Durchführung des tomographischen Verwischungsprozesses den Objektdetails aus der gewünschten, scharf abzubilden Schicht die richtigen Bildinhalte zuzuordnen und die Lage der gewünschten Schicht der Patientenbewegung nachzuführen. Die Größenkorrektur dient ausschließlich dazu, die in den Einzelbildern erfassten Bildinhalte zur Erzeugung der tomosynthetischen Bildrekonstruktion ortsgenau auswerten zu können. Die sehr aufwendigen Verfahren der tomosynthetischen Bildrekonstruktion sind für die Erstellung von Panoramaschichtaufnahmen nicht erforderlich.

Aus der EP-A-0 000 079 ist ein Röntgengerät bekannt, bei dem das massstabsgerechte Verhältnis zwischen den einzelnen Zähnen dadurch erreicht wird, dass die Geschwindigkeit mit der sich der Fokus entlang der Fokalkurve bewegt, an die jeweilige Position angepasst wird.

Ausgehend von den bisher bekannten Abhilfemöglichkeiten zur Korrektur des vergrößerungsfaktors liegt der Erfindung die Aufgabe zugrunde, ein Verfahren bereitzustellen, welches eine besser auswertbare Abbildung des Objekts in der Bildebene bereitstellt.

### Darstellung der Erfindung

Erfindungsgemäß wird diese Aufgabe durch das Verfahren gemäß Anspruch 1 gelöst. -

Das erfindungsgemäße Verfahren geht mit einer Vielzahl von Vorteilen einher. Die Aussagefähigkeit von digitalen Röntgenaufnahmen kann mit dem Verfahren erheblich verbessert werden, da-geometrische Verhältnisse aufeinander abgestimmt werden können und bei Rückrechnungen und durch Schätzung einfließende Unsicherheitsfaktoren ausgeschlossen sind. Mittels der Korrektur des Vergrö-ßerungsfaktors beispielsweise auf die in der Objektebene herrschende maßstabsgetreue Größe von 1:1, kann ein addiertes Gesamtbild in mehreren Schichtlagen erzeugt und auf dem Bildschirm in vielfältigen Darstellungen wiedergegeben werden.

In weiterer Ausgestaltung des erfindungsgemäßen Verfahrens kann eine digitale Abbildung in der Bildebene von einem Sensor mit in Zeilen und Spalten angeordneten Bildpunkte erzeugenden Pixeln aufgenommen werden. Dabei erfolgt der Aufbau der digitalen Abbildung während der Drehung eines Fokus' um ein Zentrum durch spaltenweises Auslesen der Pixel aus dem Sensor. Während der Drehung des Fokus' um das Zentrum entlang der Fokalkurve werden den Bildpunkten des aufgenommenen Objekts die Koordinaten einer definierten Schichtlage zugeordnet. Diese Zuordnung von Objekt und Schichtlage und/oder die Korrekturwerte werden vorzugsweise jeweils abgespeichert; auf diese Information kann dann wieder zugegriffen werden.

Eine Korrektur des VergröQerungsfaktors erfolgt vorteilhafterweise dann dergestalt, dass jeder Bildpunkt für eine vorgegebene Schichtlage in seiner Höhe auf die Höhe umgerechnet wird, die in der Fokalebene, also beispielsweise des abzubildenden Ober- oder Unterkiefers, vorliegt. Dabei können die Bildpunkte für jede vorgegebene Schichtlage auch in der Breite umgerechnet werden.

Die auf die Höhe der Objektebene, also der Fokalebene, umgerechneten Bildpunkte können dann so korrigiert in der Bildebene abgebildet werden und beispielsweise auf einem Monitor eines Bildschirmes im Maßstab 1:1 maßstabsgerecht ohne Verzerrung wahrnehmbar gemacht werden. Die auf diese durch Umrechnung der Bildpunkte erhaltene maßstabsgerechte Abbildung ohne Verzerrung kann anschließend in vielfältiger Weise weiterverarbeitet werden.

Vorteilhafterweise wird aus allen umgerechneten Schichtlagen eine Gesamtabbildung berechnet, die insbesondere so ausgeführt sein kann, dass das Objekt in der korrigierten Aufnahme in der Größe, in der es in der Fokalebene vorliegt, abgebildet wird.

Besonders vorteilhaft ist das Verfahren dann, wenn in einer Schichtlage der Röntgenschichtaufnahme Bildpunkte mit unterschiedlichen Abständen erzeugt werden und/oder vorhanden sind, so dass innerhalb einer Schichtlage unterschiedliche Tiefenschärfen hergestellt werden können und dabei die unterschiedlichen Abbildungsmaßstäbe berücksichtigt werden können. Das grundsätzliche Prinzip zur Erstellung von Schichtaufnahmen mit unterschiedlicher Tiefeninformation bzw. Tiefenschärfe ist in der DE 197 33 338 A1 offenbart.

Das erfindungsgemäße Korrekturverfahren ist besonders vorteilhaft bei einem Röntgengerät zur Aufnahme digitaler Röntgenaufnahmen einsetzbar, bei dem ein Fokus entlang einer Fokalkurve um ein Zentrum bewegbar ist, wobei ein Sensor in der Bildebene liegt, und wobei eine Abbildungsgröße der in- der Bildebene liegenden Bildpunkte für vorgegebene Schichtlagen auf eine korrigierte Abbildungsgröße der Objekte in der Fokalkurve umgerechnet, wozu Mittel vorhanden sind, in denen die zu den Bildpunkten einer Schichtlage zugehörigen Korrekturfaktoren abgespeichert sind. Dadurch ist es möglich, die erfindungsgemäße Korrektur in Abhängigkeit der Schichtlage durchzuführen.

Widerrum in bezug auf die DE 197 33 338 ist ein Röntgengerät so weitergebildet, dass zur nachträglichen Bestimmung der scharfen Schicht und/oder der Tiefenschärfe der Sensor aus einer Vielzahl von schmalen auslesbaren Zonen besteht, die tomographische Bildinformationen hoher Tiefenschärfe und vorbestimmter Schichtlage erzeugen, wobei diese Bildinformationen einzeln ausgelesen und in einer Bildverarbeitungseinheit für ein Bild geringer Tiefenschärfe verrechnet werden, wobei der Abstand dieses Bildes zur der Fokalebene durch Veränderung des Ortsversatzes der einzelnen tomographischen Bilder mit Hilfe einer auf die Bildverarbeitung einwirkenden Eingabeeinrichtung bestimmbar ist.

### Kurzbeschreibung der Zeichnung

Anhand einer Zeichnung sei die Erfindung nachstehend im Detail erläutert.

Es zeigt:
- Figur 1: den Strahlengang vom Fokus durch die Fokalebene auf eine Sensorebene,
- Figur 2a: eine vollständige Schichtlagenaufnahme,
- Figur 2b: eine Detailansicht mit unterschiedlichen scharfen Schichten entlang der Fokalebene
- Figur 3: eine schematische Auswerteeinheit zur Korrektur des Vergrößerungsfaktors.

### Ausführungsbeispiel

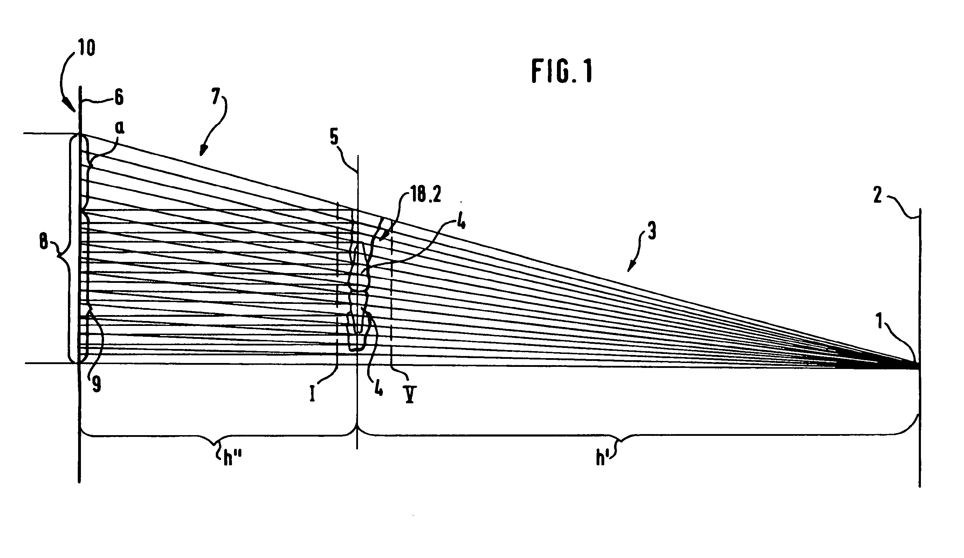

Figur 1 zeigt den Strahlengang ausgehend vom Fokus durch die Fokalebene auf eine Sensorebene.

Aus der schematischen Skizze geht hervor, dass eine Fokus 1 beispielsweise eine Röntgenquelle, ein Röntgenstrahlenbündel 3 aussendet. Das Strahlenbündel 3 fächert sich wie in Figur 1 dargestellt auf und durchdringt ein Objekt 4 in einer Fokalebene 5, die sich im bekannten Abstand h' vom Fokus 1 befindet. Im vorliegenden Falle gemäß Figur 1 liegen in der Fokalebene 5 die Zähne des Ober- und Unterkiefers, die von dem Strahlenbündel 3 durchdrungen werden.

Die Fokalebene 5 kann zwischen den Positionen I und V mit veränderten Abständen h' und h" zu liegen kommen, wenn das selbe Objekt mehrmals durchstrahlt wird oder andere Maßnahmen ergriffen werden, um die Tiefenschärfe zu beeinflussen, wir beispielsweise in der DE 197 33 338 A1 offenbart ist, aber auch durch Mehrfachaufnahmen bewirkt werden kann.

Das durch Absorption beeinflusste Strahlenbündel 7 fällt in der Empfangs- oder Bildebene 6 auf einen dort angeordneten Sensor 10. Aufgrund des Strahlensatzes werden die Objekte der Fokalebene 5 statt in ihrer maßstabsgerechten (M 1:1) Größe 9 mit einem Vergrößerungsfaktor, der sich bei dentalen Panorama-Schichtaufnahmen zwischen 1,05 und 1,24 bewegt, mit der Höhe 8 in der Bildebene 6 abgebildet.

Die strahlungsempfindliche Oberfläche (Bildebene 6) des Sensors 10 ist im bekannten Abstand von h" von der Fokalebene 5 angeordnet. Mit a ist die Verzerrung des Abbildes in vertikale Richtung gekennzeichnet, die die vertikale Erstreckung der Abbildung nicht maßstabsgerecht erscheinen lässt.

Der in der Bildebene 6 angeordneter Sensor 10 weist auf seiner Oberfläche strahlungsempfindliche Pixel auf. Die Pixel sind jeweils symmetrisch zur Mitte der strahlungsempfindlichen Fläche verteilt angeordnet. Die strahlungsempfindliche Oberfläche des Sensors kann 66 und mehr sich in vertikaler Richtung erstreckende Spalten aufweisen, von denen jede wiederum in 1500 und mehr Zeilen unterteilt sein kann. Ein Pixel lässt sich dabei genau einer Zeile und einer Spalte zuordnen, wodurch jedes Pixel auf der Oberfläche des Sensors eindeutig adressierbar ist.

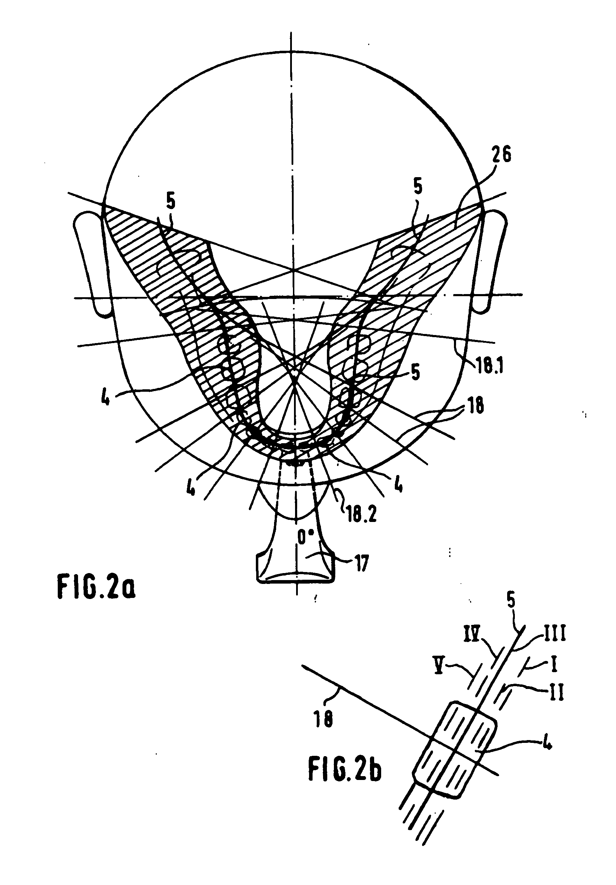

Pro TDI-Impuls, mit der die Röntgenaufnahme gemacht wird, wird eine komplette Pixelspalte aus dem beispielsweise als Schieberegister funktionierenden Sensor 10 per Signalausgang ausgelesen, während gleichzeitig die dadurch in Aufnahmerichtung nächstliegende Spalte wieder neu belichtet wird; somit entsteht die Röntgenaufnahme spaltenweise, während gleichzeitig die Schichtlage identifiziert und abgespeichert wird, für welche die aufgenommene spaltenweise Pixelspalte gerade ausgelesen wurde. So wird eine Zuordnung zwischen Pixelspalte, Sensor und zugehöriger Schichtlage - beispielsweise im Ober- oder Unterkiefer - sichergestellt (vgl. Figur 2a).

In Figur 2a ist eine vollständige Schichtlagenaufnahme mit stilisierter Einzeichnung des scharf abgebildeten Kieferbereiches dargestellt, bezeichnet als Schichtdickenverlauf 26.

Während des Aufnahmezyklus der Röntgenaufnahme, d.h. des Umlaufs des Fokus 1 um einen Drehpunkt wird der gesamte, durchstrahlte, die Fokalebene 5 enthaltende Kiefer abgefahren und jede hier durch Striche 18 dargestellte Schichtlage abgebildet. Mit 18.1 ist eine Schichtlage bezeichnet, die im Backenzahnbereich liegt, in dem der Kiefer etwas dicker ausgebildet ist. Verglichen mit der Schichtlage 18.1 ist die den Schneidezahnbereich an Ober- und Unterkiefer durchdringende Schichtlage 18.2 wesentlich dünner ausgeprägt. Die Schichtlagen 18 schließen die Fokalebene 5 jeweils ein und stellen die Bereiche dar, in denen die Objekte 4 scharf abgebildet sind. Die Schichtlagendicke kann je nach Kieferanatomie dicker oder dünner sein, ist aber bei der Erstellung des Röntgenaufnahme bekannt.

Die Dicke der Schichtlage hängt nicht zuletzt damit zusammen, wie groß der Abstand h" von der Fokalebene 5 zu dem Sensor ist. Aufgrund der Kieferanatomie verändert sich während der Aufnahme des Röntgenbildes der Abstand h' zwischen Fokus 1 und dem in der Fokalebene 5 liegenden Objekt 4. Dadurch ändert sich auch der Abstand h" zwischen Objekt 4 und der Bildempfangsebene 6 während der Aufnahme des Röntgenbildes. Der Abstand h" ist im Backenzahnbereich gering, im Frontzahnbereich hingegen deutlich größer.

In Fig. 2b sind verschiedene scharfe Schichten I bis V durch das Objekt 4 dargestellt, die jeweils der Fokalebene 5 mit entsprechendem Versatz in Richtung der Schichtlage 18 entsprechen. Hierdurch werden Tiefeninformationen gewonnen, die beim Übergang von einer scharfen Schicht I zur nächsten scharfen Schicht II usw. jeweils in ihrer Größe korrigiert werden, sodass trotz der Strahlaufweitung stets ein gleichgroßes Vergleichsschnittbild des Objekts 4 mit jeweils unterschiedlicher scharfer Schicht vorhanden ist.

Um während des Aufbaus der Röntgenaufnahme eine definierte, die Aussagefähigkeit des Röntgenbildes erhöhende Position von Ober- und Unterkiefer zueinander und zum Fokus 5 und Sensor 10 zu gewährleisten, ist ein Aufbiss 17 eingezeichnet, welcher Ober- und Unterkiefer des Patienten zueinander ausrichtet.

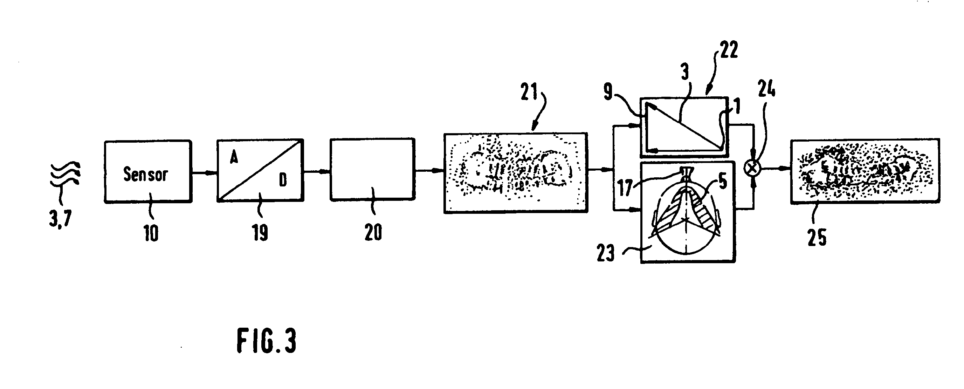

In Figur 3 ist ein Auswertablauf dargestellt, welcher zur Korrektur des Vergrößerungsfaktors in der Röntgenabbildung dient.

Das Strahlenbündel 3, 7 trifft nach Durchdringung der in der Fokalebene 5 liegenden Objekte auf den Pixeln des Sensors auf, welcher die erhaltenen Bilddaten spaltenweise zur Digitalisierung einem A/D-Wandler 19 zuführt. Im A/D-Wandler 19 werden die analog vorliegenden Bilddaten für jedes beleuchtete Pixel digitalisiert und einem Speicher 20 zugeführt. Aus dem per Umlauf erhaltenen spaltenweise anfallenden Bilddaten wird das gesamte Röntgenbild 21 aufgebaut, welches um einen Objektvergrößerungsfaktor zwischen 1,05:1 und 1,24:1 überhöht ist.

Da in einer Speichereinheit 22 jedes Pixel/Bildpunkt mit seinen Koordinaten x, y adressierbar ist, kann die Höhe 9 des Pixels für die Objektebene mit den dazugehörigen Vergrößerungsfaktoren, die aus den Abständen h', h" bekannt sind, vorgewählt werden, so dass das außenliegende Pixel nicht seine gestreckte Höhe 8 in der Empfangsebene 6 sondern rechnerisch die maßstabsgerechte Höhe 9 annimmt.

In einem Schichtlagespeicher 23 sind während der Röntgenaufnahme alle durchstrahlten Schichtlagen 18 abgespeichert, die sich nunmehr, beispielsweise pixelspaltenweise, mit den korrigierten Höhen 9 der Pixel verknüpfen lassen. Aus der Verknüpfung der korrigierten Pixelhöhe 9 mit der jeweiligen Schichtlage 18 lässt sich eine Abbildung 25 aufbauen, die in Objektgröße im Maßstab 1:1 ausgegeben werden kann. In der Recheneinheit 24 erfolgt ein Neuaufbau der digitalisierten Röntgenaufnahme, wobei durch die Bestimmung der Pixelhöhe 9 für jede Pixelspalte auch entlang der Fokalebene unterschiedlichen Vergrößerungsfaktoren Rechnung getragen werden kann. Alle auf den Maßstab von 1:1 umgerechneten Schichtlagen 18 können zu einem Gesamtbild addiert und auch bei Bedarf aus diesem subtrahiert werden.

Neben der Umrechnung der Schichtlagen 18 auf den Maßstab 1:1 können diese auch in beliebige andere Maßstäbe umgerechnet werden. In der Auswerteeinheit 24 gemäß Figur 3 lassen sich mithin die spaltenweise vorliegenden Bildinformationen mit den dazugehörigen Vergrößerungsfaktoren so korrigieren, dass sowohl eine Röntgenaufnahme im Maßstab 1:1 als auch eine bewusst in Breite und/oder Höhe verzerrte Röntgenbildaufnahme 25 entsteht.

Die umgerechneten Schichten 18 können auf einem Monitor - in vielfältiger Weise dargestellt werden, auch eine Ausgabe auf einem Drucker oder auf einem anderen Medium ist denkbar.

Neben der Anwendung des erfindungsgemäßen Verfahrens auf mit Sensoren erfassbare digitale Aufnahmen lassen sich auch bei Filmaufnahmen, die in digitale Form überführt wurden, die Fokalparameter dem digitalisierten Bild je nach Anwendungszweck des Anwenders zuordnen und aufbereiten.

### Bezugszeichenliste

- 1: Fokus
- 2: Fokusebene
- 3: Strahlenbündel
- 4: Objekt
- 5: Fokalebene, Maßstab 1:1
- 6: Empfangsebene
- 7: Strahlenbündel
- 8: Abbildungsgröße Bildebene
- 9: korrigierte Abbildungsgröße
- 10: Sensor
- 17: Aufbiss
- 18: durchstrahlte Schichtlage
- 18.1: erste Schichtdicke in Durchstrahlungsrichtung
- 18.2: zweite Schichtdicke in Durchstrahlungsrichtung
- 19: A/D - Wandler
- 20: Speicher
- 21: digitales Röntgenbild Bildebene
- 22: Speichereinheit für die Pixeldefinition (x, y)
- 23: Schichtlagenspeicher
- 24: Recheneinheit
- 25: Röntgenaufnahme in Objektgröße 1:1
- 26: Schichtdickenverlauf

## Patentansprüche

1. Verfahren zur Erstellung und Wiedergabe eines Schichtbildes (25) eines Objekts (4) aus einer Rtintgenschichtaufnahme (21), bei der ein Fokus (1) der verwendeten Strahlung in einem bekannten Abstand (h') zu einer im Bereich des Objekts (4) liegenden Fokalebene (5) und die Fokalebene (5) wiederum in einem bekannten Abstand (h") zu einem in der Bildebene (6) angeordneten, eine Vielzahl von Bildpunkten aufweisenden Strahlenempfänger (10) angeordnet ist, so dass eine Schichtlage (18) durchstrahlt wird, aufweisend die nachfolgenden Verfahrensschritte:
- Erstellen einer digitalen Röntgenschichtaufnahme (21) oder Abrufen einer digitalen Röntgenschichtaufnahme (21) aus einem Speicher oder Digitalisieren einer analogen Röntgenschichtaufnahme (21);
- Definieren eines mehrere Bildpunkte umfassenden Korrekturbereiches in der Röntgenschichtaufnahme (21) ;
- Zuordnung der Schichtlage (18, 18.1, 18.2) mit den bekannten Abständen (h', h") zu dem Bildpunkt des aufgenommenen Objekts (4) und
- Zuordnung eines Korrekturfaktors zu den entsprechenden Bildpunkten eines Korrekturbereichs unter Berücksichtigung der Schichtlage (18, 18.1, 18.2) mit den bekannten Abständen (h', h");
- Umrechnen jedes Bildpunktes der Bildebene (6) des Korrekturbereichs mit dem Korrekturfaktor;
- Anordnen jedes aus der Bildebene (6) umgerechneten Bildpunkts in einer korrigierten Aufnahme (25).

2. Verfahren gemäß Anspruch 1, **dadurch gekennzeichnet, dass** eine Abbildung (21) in der Bildebene (6) von einem Sensor mit Bildpunkte erzeugenden Pixeln, die in Zeilen und Spalten angeordnet sind, aufgenommen wird, dass der Aufbau der Abbildung - (21) während der Drehung des Fokus (1) um ein Zentrum durch spaltenweises Auslesen der Pixel aus einem Sensor (10) erfolgt und dass während der Drehung des Fokus (1) um das Zentrum entlang der Fokalebene (5) die Koordinaten der Schichtlage (18) und/oder die Korrekturwerte den Bildpunkten des jeweils aufgenommenen Objekts (4) zugeordnet werden.

3. Verfahren gemäß Anspruch 1, **dadurch gekennzeichnet, daß** jeder Bildpunkt für eine vorgegebene Schichtlage (18) in der Höhe (8) auf die Höhe (9) der Fokalebene (5) und/oder jeder Bildpunkt für eine vorgegebene Schichtlage (18) in der Breite umgerechnet wird.

4. Verfahren gemäß Anspruch 3, **dadurch gekennzeichnet, daß** jeder Bildpunkt in der Bildebene (6) abgebildet wird.

5. Verfahren gemäß Anspruch 1, **dadurch gekennzeichnet, daß** das für die ausgewählte Schichtlage (18) umgerechnete aus Bildpunkten bestehende Bild wahrnehmbar gemacht wird.

6. Verfahren gemäß Anspruch 1, **dadurch gekennzeichnet, daß** aus allen umgerechneten Schichtlagen (18) eine Gesamtabbildung (25) berechnet wird.

7. Verfahren gemäß Anspruch 1, **dadurch gekennzeichnet, dass** das Objekt (4) in der korrigierten Aufnahme (25) in der Größe, in der es in der Fokalebene (5) vorliegt, abgebildet wird.

8. Verfahren gemäß Anspruch 1, **dadurch gekennzeichnet, dass** in einer Schichtlage der Röntgenschichtaufnahme Bildpunkte mit unterschiedlichen Abständen (h', h") erzeugt werden und/oder vorhanden sind.

9. Röntgengerät zur Erstellung digitaler Röntgenaufnahmen von Objekten (4), bei der ein Fokus (1) der verwendeten Strahlung in einem bekannten Abstand (h') zu einer im Bereich des Objekts (4) liegenden Fokalebene (5) und die Fokalebene (5) wiederum in einem bekannten Abstand (h") zu einem in der Bildebene (6) angeordneten Strahlenempfänger (10) angeordnet ist, **dadurch gekennzeichnet, daß** eine Abbildungsgröße (8) der in der Bildebene (6) liegenden Bildpunkte für vorgegebene Schichtlagen (18) auf eine korrigierte Abbildungsgröße (9) der Objekte (4) in der Fokalebene (5) umgerechnet wird, wozu Mittel vorhanden sind, in denen die zu den Bildpunkten einer Schichtlage (18) zugehörigen Korrekturwerte abgespeichert sind.

10. Röntgengerät nach Anspruch 9, **dadurch gekennzeichnet, dass** zur nachträglichen Bestimmung der scharfen Schicht und/oder der Tiefenschärfe der Sensor aus einer Vielzahl von schmalen auslesbaren Zonen besteht, die tomographische Bildinformationen hoher Tiefenschärfe und vorbestimmter Schichtlage erzeugen, wobei diese Bildinformationen einzeln ausgelesen und in einer Bildverarbeitungseinheit (7) für ein Bild geringer Tiefenschärfe verrechnet werden, wobei der Abstand (h', h") dieses Bildes zu der Fokalebene durch Veränderung des Ortsversatzes der einzelnen tomographischen Bilder mit Hilfe einer auf die Bildverarbeitung (7) einwirkenden Eingabeeinrichtung (12) bestimmbar ist.

## Claims

1. Method for the creation and rendering of a tomographic image (25) of an object (4) from a radiological radiogram (21), whereby a focus (1) of the applied radiation is arranged at a known distance (h') from a focal plane (5) lying in the region of the object (4) and the focal plane (5) is in turn arranged at a known distance (h") from a sensor that is arranged in the image plane (6) and comprises a plurality of picture elements (10), so that a slice position (18) is transirradiated, comprising the following method steps:
- creating a digital radiological radiogram (21) or recalling a digital radiological radiogram (21) from a memory or digitalizing an analog radiological radiogram (21);
- defining a correction region comprising a plurality of picture elements in the radiological radiogram (21);
- allocating the slice position (18, 18.1, 18.2) with the known distances (h', h") to the picture element of the registered object (4) and
- allocating a correction factor to the corresponding picture elements of a correction region taking the slice position (18, 18.1, 18.2) with the known distances (h'; h") into consideration;
- converting each picture element of the image plane (6) of the correction region with the correction factor;
- arranging each converted picture element from the image plane (6) in a corrected exposure (25).

2. Method according to claim 1, **characterized in that** an image (21) in the image plane (6) is registered by a sensor with pixels that generate picture elements and that are arranged in rows and columns; **in that** the formation of the image (21) is carried out by column-by-column readout of the pixels from a sensor (10) during the rotation of the focus (1) around a center; and **in that** the coordinates of the slice position (18) and/or the correction values are allocated to the picture elements of the respectively imaged object (4) during the rotation of the focus (1) around the center along the focal plane (5).

3. Method according to claim 1, **characterized in that** each picture element for a predetermined slice position (18) is converted in height (8) to the height (9) of the focal plane (5) and/or each picture element for a predetermined slice position (18) is converted in width.

4. Method according to claim 3, **characterized in that** each picture element is displayed in the image plane (6).

5. Method according to claim 1, **characterized in that** the image that is composed of picture elements and that is converted for the selected slice position (18) is rendered observable.

6. Method according to claim 1, **characterized in that** an overall image (25) is calculated from all converted slice positions (18).

7. Method according to claim 1, **characterized in that** the object (4) is displayed in the corrected exposure (25) in the same size with which it is present in the focal plane (5).

8. Method according to claim 1, **characterized in that** picture elements having different distances (h', h") are generated and/or are present in a slice position of the radiological radiogram.

9. X-ray apparatus for creating digital radiograms of objects (4), having a focus (1) of the applied radiation with a known distance (h') to a focal plane lying in an area of the object (4) and the focal plane (5) having a known distance (h") to a sensor (10) lying in an image plane (6), **characterized in that** an image size (8) of the picture elements lying in the image plane (6) for predetermined slice positions (18) is converted onto a corrected image size (9) of the objects (4) in the focal plane (5), to which means are provided wherein the correction factors belonging to the picture elements of a slice position are stored.

10. X-ray apparatus according to claim 9, **characterized in that**, for subsequent determination of the sharp slice and/or the depth of focus, the sensor is composed of a plurality of narrow zones that can be read out, these generating tomographic image information with high depth of focus and predetermined slice position, whereby these image informations are individually read out and converted for an image with low depth of focus in an image processing unit (7), whereby the distance (h', h") of this image from the focal plane can be identified by modifying the topical offset of the individual tomographic images by means of an input device (12) that influences the image processing (7).

## Revendications

1. Procédé permettant de générer et de restituer une image de coupe (25) d'un objet (4) à partir d'une tomographie (21), dans lequel on effectue une focalisation (1) du rayonnement utilisé à une distance connue (h') d'un plan focal (5) situé dans le voisinage de l'objet (4), et le plan focal (5) étant lui-même à une distance connue (h") d'un récepteur de rayons (10) présentant une multiplicité de points d'image et disposé dans le plan image (6), de telle sorte qu'une position de couche (18) est traversée par le rayonnement, présentant les étapes suivantes du procédé :
- générer une tomographie numérique (21) ou récupérer d'une tomographie numérique (21) à partir d'une mémoire ou numériser une tomographie analogique (21) ;
- définir un domaine de correction comportant plusieurs points d'image dans la tomographie (21) ;
- attribution de la position de couche (18, 18.1, 18.2) avec les distances connues (h', h") au point d'image de l'objet (4) enregistré et
- attribution d'un facteur de correction aux points d'image correspondants d'un domaine de correction en respectant la position de couche (18, 18.1, 18.2) avec les distances connues (h', h") ;
- convertir chaque point d'image du plan image (6) du domaine de correction avec le facteur de correction ;
- disposer chaque point d'image converti depuis le plan image (6) dans un enregistrement (25) corrigé de l'image.

2. Procédé selon la revendication 1, **caractérisé en ce qu'**on enregistre une image (21) dans le plan image (6) au moyen d'un capteur avec des pixels générant les points d'image, disposés en lignes et en colonnes, **en ce que** l'élaboration de l'image (21) est réalisée pendant la rotation de la focalisation (1) autour d'un centre par lecture en colonne des pixels d'un capteur (10) et **en ce qu'**on attribue les coordonnées à la position de couche (18) et/ou les valeurs de correction aux points d'image de l'objet (4) déjà enregistré, pendant la rotation de la focalisation (1) autour du centre le long du plan focal (5).

3. Procédé selon la revendication 1, **caractérisé en ce que** chaque point d'image est converti pour une position de couche (18) donnée en hauteur (8) par rapport à la hauteur (9) du plan focal (5) et/ou que chaque point d'image est converti pour une position de couche (18) donnée en largeur.

4. Procédé selon la revendication 3, **caractérisé en ce que** chaque point d'image est reproduit dans le plan image (6).

5. Procédé selon la revendication 1, **caractérisé en ce que** l'image convertie pour la position de couche choisie (18) et constituée de points d'image, est rendue discernable.

6. Procédé selon la revendication 1, **caractérisé en ce qu'**on calcule une image globale (25) à partir de toutes les positions de couche (18) converties.

7. Procédé selon la revendication 1, **caractérisé en ce qu'**on reproduise l'objet (4) dans l'enregistrement corrigé (25) de l'image à la taille à laquelle il existe dans le plan focal (5).

8. Procédé selon la revendication 1, **caractérisé en ce que** dans une position de couche de la tomographie, on génère et/ou il existe des points d'image ayant des distances différentes (h', h").

9. Appareil de radiographie permettant de générer des images numériques de radiographie d'objets (4), dans lesquelles on effectue une focalisation (1) du rayonnement utilisé à une distance connue (h') d'un plan focal (5) situé dans le domaine de l'objet (4), et le plan focal (5) étant lui-même à une distance connue (h") d'un récepteur de rayons (10) disposé dans le plan image (6), **caractérisé en ce qu'**une taille de l'image (8) des points de l'image situés dans le plan image (6) pour des positions de couche (18) données est convertie en une taille de l'image corrigée (9) des objets (4) dans le plan focal (5) ce pour quoi on dispose de moyens permettant d'enregistrer les valeurs de corrections affectées aux points de l'image d'une position de couche (18).

10. Appareil de radiographie selon la revendication 9, **caractérisé en ce que** pour la définition ultérieure de la couche mise au point et/ou de la profondeur de champ, le capteur est constitué d'une multiplicité de zones lisibles étroites qui génèrent des informations d'images tomographiques de grande profondeur de champ et de position de couche définie, ces informations d'image étant lues individuellement et calculées dans une unité de traitement d'image (7) pour une image de profondeur de champ réduite, la distance (h', h") de cette image au plan focal pouvant être définie en modifiant le déplacement spatial des images tomographiques individuelles à l'aide d'une installation de saisie (12) agissant sur le traitement d'image (7).
